# EUROPEAN PATENT APPLICATION

(11) **EP 1 962 106 A1**
(43) Date of publication of application: **27.08.2008**
(21) Application number: 08002629.7
(22) Date of filing: 13.02.2008
(51) Int. Cl.: G01S 15/89, G01S 7/521

(54) **Ultrasound system**

(30) Priority: 14.02.2007 KR 20070015221
(71) Applicant: MEDISON CO., LTD., Kangwon-do 250-870 (KR)
(72) Inventor: Kang, Hak Il, Gangnam-gu Seoul 135-280 (KR)
(74) Representative: Schmid, Wolfgang

(57) **Abstract**

The present invention is directed to an ultrasound system, which includes a probe to transmit ultrasound signals to a target object and receive ultrasound echo signals from the target object. The probe has an array transducer having multiple transducer elements for generating the ultrasound signals and converting the ultrasound echo signals into electrical signals; and an input unit to receive a swing speed input and form setup information according to the swing speed input. The ultrasound system further includes a body to form an ultrasound image based on the electric signals. The body includes a control unit for generating a control signal in response to the setup information. The probe further has an operation unit to swing the array transducer at a swing speed indicated by the control signal.

## Description

### ULTRASOUND SYSTEM

The present application claims priority from Korean Patent Application No. 10-2007-0015221 filed on February 14, 2007, the entire subject matter of which is incorporated herein by reference.

### BACKGROUND OF THE INVENTION

### [Technical Field]

The present invention relates to ultrasound systems, and more particularly to an ultrasound system for adjusting the resolution of an ultrasound image with an input unit mounted on a probe.

### [Background Art]

The ultrasound system has become an important and popular diagnostic tool due to its non-invasive and non-destructive nature. Modem high-performance ultrasound imaging diagnostic systems and techniques are commonly used to produce two- or three-dimensional images of internal features of patients

The ultrasound system generally uses a probe containing an array of transducer elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce an ultrasound image data.

The probe may be classified according to various conditions such as the number of transducer elements, an array type of transducer elements, the shape of array transducer, etc. The probe may be classified into a single-element probe and a multi-element probe according to the number of transducer elements. Also, the probe may be classified into a 1-dimensional array probe in which the transducer elements are arrayed in a single axis and a 2-dimensional array probe in which the transducer elements are arrayed in multiple axes.

When the 2-dimensional array probe is used, only a 2-dimensional ultrasound image corresponding to a plane in front of the 2-dimensional array probe in a target object may be obtained. However, it is limited to utilizing the ultrasound image for accurate diagnosis. Also, with the use of the 2-dimensioal array probe, it is difficult to form a 3-dimensional ultrasound image of a target object such as a fetus and a moving image of the fetus" Thus, a 3-dimensional probe has been used to provide a 3-dimensional ultrasound image containing clinical information such as spatial information and anatomical information, which cannot be provided from the 2-dimensional ultrasound image. The 3-dimensional probe includes an operation unit on which a motor is installed for swinging an array axis of the array transducer within a predetermined angle range.

In the conventional ultrasound system, the resolution of the 3-dimensional ultrasound image has been adjusted by controlling the swing speed of the array axis. The swing speed of the array transducer can be controlled according to setup information, which is inputted through a control panel provided in a body of the ultrasound system from a user. Thus, the user has to inevitably suffer inconvenience since he/she has to manipulate the control panel in order to adjust the resolution of the ultrasound image while handling the 3-dimensional probe.

### BRIEF DESCRIPTION OF THE DRAWINGS

FIG. 1 is a block diagram showing an ultrasound system constructed in accordance with one embodiment of the present invention.

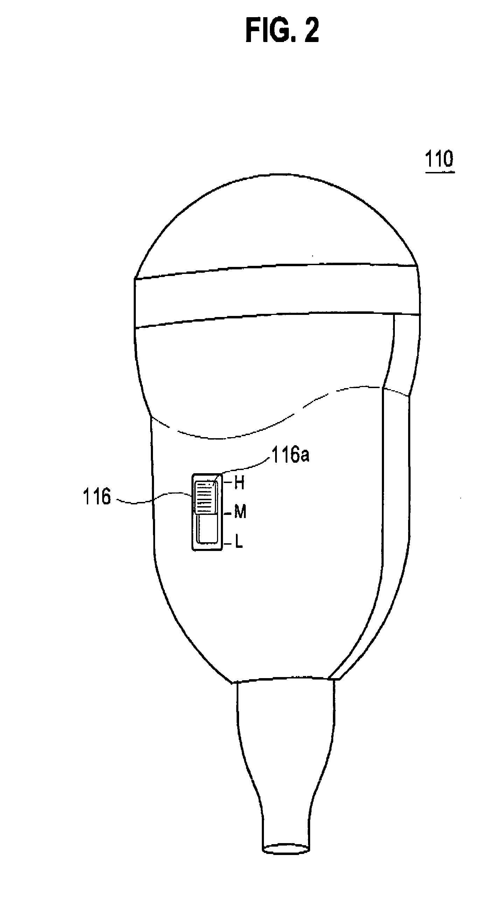

FIG. 2 is a schematic diagram showing a 3-dimensional probe having an input unit in accordance with one embodiment of the present invention.

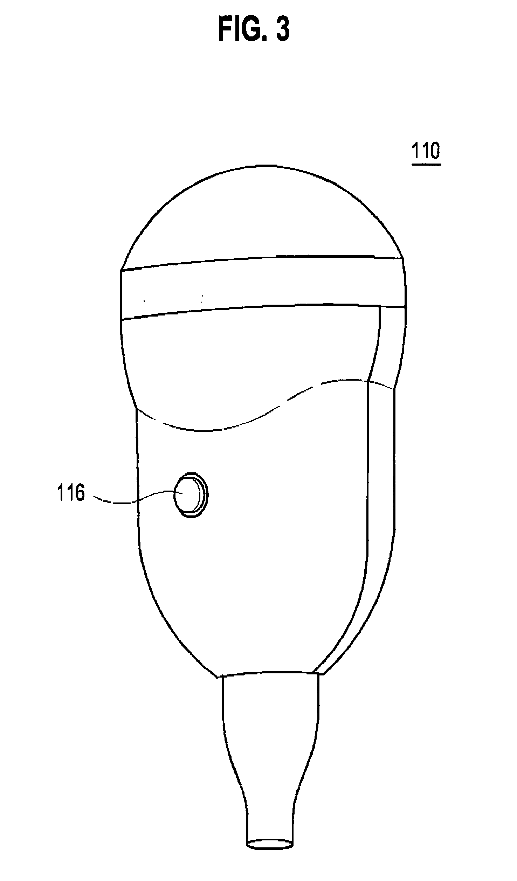

FIG. 3 is a schematic diagram showing a 3-dimensional probe having an input unit in accordance with another embodiment of the present invention.

### DETAILED DESCRIPTION OF THE INVENTION

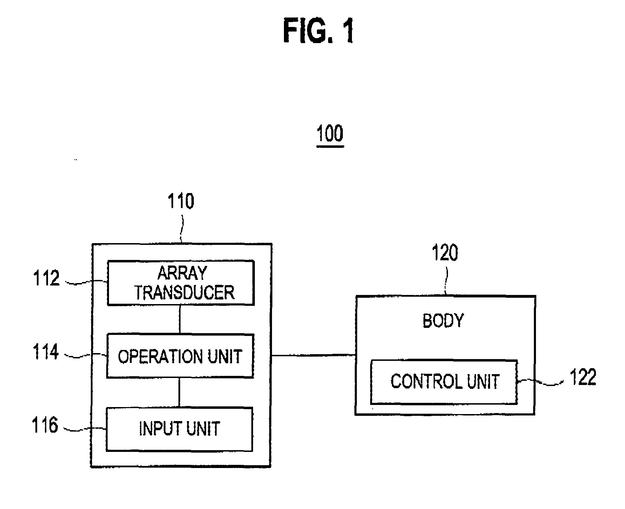

FIG. 1 is a block diagram showing an ultrasound system constructed in accordance with one embodiment of the present invention. As shown in Fig. 1, the ultrasound system 100 includes a probe 110 and a body 120 The body 120 includes a control unit 122 for controlling the entire operations of the ultrasound system 100.

The probe 110 includes an array transducer 112 comprising multiple transducer elements, an operation unit 114 and an input unit 116. The array transducer 112 generates ultrasound signals to be transmitted to a target object and receives ultrasound signals reflected from the target object. The operation unit 114 includes a motor in order to swing the array transducer within a predetermined angle range under the control of the control unit 122 included in the body 120, thereby moving a scanning plane in the target object.

The input unit 116 receives a swing speed input and forms setup information according to the swing speed input. Resolution of the ultrasound image may be adjusted according to the swing speed of the array transducer. The inputted setup information is transmitted to the control unit 122 in the body 120 via wired or wireless means.

The input unit 116 may include a variable resistor in accordance with one embodiment of the present invention. The variable resistor may be a slide type of variable resistor whose resistance varies by moving a knob 116a installed on the input unit 116 up and down (or right and left). The resistance selected by moving the knob 116a may be used to form the setup information" The speed of the motor in the operation unit 114 may be adjusted to a low speed L, a middle speed M or a high speed H according to the selected resistance in accordance with one embodiment of the present invention. The low speed is a speed, which is relatively lower than a typical speed of the motor in the operation unit 114. The low speed may allow the slow swinging of the transducer elements so that the resolution of the ultrasound image may be increased The high speed is a speed, which is relatively higher than the typical speed of the motor in the operation unit 114. The high speed may enable the array transducer to rapidly swing so that a frame rate may be increased" At the high speed, the resolution of the ultrasound image may be decreased. The middle speed corresponds to the typical speed of the motor in the operation unit 114.

In accordance with another embodiment of the present invention, the input unit 116 may include a button receiving the swing speed input to form the setup information. The speed of the motor in the operation unit 114 may be controlled according to the number of times the button is pressed. That is, whenever the button is pushed, the setup information is iteratively changed to information upon a low speed, a middle speed and a high speed.

The body 120 may further include a beam former, an amplification unit, a processor and a display unit. The beam former may be operable to form a transmission pattern of transmission pulse signals such that ultrasound signals generated in the transducer elements in response to the transmission pulse signals can be focused on a focal point in the target object. Further, the beam former may be operable to focus signals outputted from the transducer elements in response to ultrasound echo signals to thereby form a reception beam. The amplification unit may be operable to amplify the reception beam and adjust gain of the amplified reception beam. The processor may be operable to form an ultrasound image based on the amplified and gain-adjusted reception beam. The display unit may display the ultrasound image. Further, the body may include a communication module for wirelessly transmitting and receiving data with the probe 110.

The control unit 122 included in the body 120 transmits a control signal corresponding to the setup information received from the input unit 116, The speed of the motor in the operation unit 114 may be controlled by the control signal so that the swing speed of the transducer elements may be adjusted.

Although embodiments have been described with an input unit as the slide type of variable resistor and button, it should be understood that any type of input unit capable of receiving the swing speed input may be used.

As described above, the swing speed of the transducer elements in the probe can be adjusted by using the input unit mounted on the probe. Accordingly, the resolution of the ultrasound image can be easily adjusted in the ultrasound system.

In accordance with one embodiment of the present invention, there is provided an ultrasound system, comprising a probe to transmit ultrasound signals to a target object and receive ultrasound echo signals from the target object. The probe includes the following an array transducer having multiple transducer elements for generating the ultrasound signals and converting the ultrasound echo signals into electrical signals; an input unit to receive a swing speed input and form setup information according to the swing speed input; and a body to form an ultrasound image based on the electric signals, said body including a control unit for generating a control signal in response to the setup information, said probe further including an operation unit to swing the array transducer at a swing speed indicated by the control signal.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment- Further, when a particular feature, structure, or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a probe to transmit ultrasound signals to a target object and receive ultrasound echo signals from the target object, wherein the probe includes:
an array transducer having multiple transducer elements to generate the ultrasound signals and convert the ultrasound echo signals into electrical signals; and
an input unit to receive a swing speed input and form setup information according to the swing speed input; and
a body to form an ultrasound image based on the electric signals, said body including a control unit for generating a control signal in response to the setup information,
said probe further including an operation unit to swing the array transducer at a swing speed indicated by the control signal.

2. The ultrasound system of Claim 1, wherein the input unit includes a slide type of variable resister for selecting one of plural resistances.

3. The ultrasound system of Claim 1, wherein the input unit includes a button.
